# EUROPEAN PATENT APPLICATION

(11) **EP 4 085 931 A1**
(43) Date of publication of application: **09.11.2022**
(21) Application number: 20909741.9
(22) Date of filing: 31.12.2020
(51) Int. Cl.: A61K 48/00, C12N 15/00

(54) **NEW METHOD FOR TARGETED EDITING OF RNA**

(30) Priority: 31.12.2019 WO PCT/CN2019/130558
(71) Applicant: EdiGene Therapeutics (Beijing) Inc., Beijing 102206 (CN)
(72) Inventor: YUAN, Pengfei, Beijing 102206 (CN); YI, Zexuan, Beijing 102206 (CN); LIU, Nengyin, Beijing 102206 (CN)
(74) Representative: Lavoix
(86) International application number: PCT/CN2020/142218
(87) International publication number: WO 2021/136520

(57) **Abstract**

Provided is a method for deaminating targeted cytosine in a target RNA of a cell. The method comprises: introducing, into the cell, a modified adenosine deaminase protein or a catalytic domain thereof, or a construct expressing the modified adenosine deaminase protein or the catalytic domain thereof, and a construct comprising an arRNA oligonucleotide that recruits the modified adenosine deaminase protein or the catalytic domain thereof to the target RNA or expressing arRNA. Further provided are an engineered composition or system for RNA editing, and a use of treating a disease by using the engineered composition or system to correct a T-C mutation.

## Description

### FIELD OF THE INVENTION

The present application belongs to the field of gene editing therapies, and specifically, the present application creates a method for targeted editing of RNA named as C to U Specific Programmable Editing of RNA (CUSPER), it includes using a CUSPER technology to perform precise site editing on RNA from C to U bases, and may be used to treat a disease caused by a T-to-C mutation.

### BACKGROUND OF THE INVENTION

In recent years, genome editing technologies led by Clustered regularly interspaced short palindromic repeats (CRISPR) are developed rapidly, and have a profound impact on many fields of biology and medicine. Many science researchers and biotech companies also devote to bring this technology to clinical. In September 2019, Professor Deng Hongkui of Peking University and collaborators publish an article 11 that firstly reports results of a clinical trial of using the CRISPR technology to edit a stem cell and infusing it back into a patient, to treat acquired immune deficiency syndrome (AIDS) and leukemia, and it makes a great contribution to transformation of the CRISPR technology in the direction of gene therapy.

Although the CRISPR technology has a great application prospect, it also has a series of defects, so that the transformation of this technology from a scientific research stage to a clinical treatment application is difficult. One of problems is a core role enzyme that the CRISPR technology uses: Cas9. A CRISPR-based DNA editing technology requires exogenous expression of Cas9 or other nucleases with similar functions, thereby the following problems are caused. Firstly, the nucleases that require the exogenous expression typically have the relatively large molecular weights, and this sharply reduces the efficiency of delivering it into a body by a virus vector. Secondly, due to the exogenous expression of the nucleases, this method has a potential nuclease off-target possibility, and this may lead to a potential carcinogenic risk in its application. Finally, exogenously expressed Cas9 and other similar nucleases are found in bacteria, and do not naturally exist in humans or mammals, this makes it possible to cause an immune response in the patient body. On the one hand, this may cause damage to the patient himself, and on the other hand, the exogenously expressed nuclease may also be neutralized, thereby the original activity is lost, and the therapeutic effect is affected.

In 2017, Professor Zhang Feng of Massachusetts Institute of Technology (MIT) and his research group report an RNA editing technology named as RNA Editing for Programmable A to I Replacement (REPAIR), which may achieve A to I editing 2 of targeting a target RNA by exogenously expressing a Cas13-ADAR fusion protein and a single guide RNA (sgRNA), but this method, like the CRISPR technology, still requires the expression of a foreign protein. The problems caused by the expression of the foreign protein may not be solved.

In January 2019, Thorsten Stafforst's research group reports a RNA single-base editing technology named as recruiting endogenous ADAR to specific trans for oligonucleotide-mediated RNA editing (RESTORE) (Merkle et al., 2019). RESTORE may get rid of the dependence on the foreign proteins, but the RESTORE technology needs to have the higher editing efficiency in the presence of IFN-γ, and IFN-γ is a key factor 9 in determining the development and severity of autoimmunity, so the application of this technology in the medical field is greatly reduced. On the other hand, a section of a guide RNA is also used in the RESTORE technology, and the guide RNA used is a chemically synthesized oligonucleotide, and the synthesized oligonucleotide needs to artificially introduce a large number of chemical modifications to guarantee its stability. In these chemical modifications, some of which are non-natural modifications, so that the oligonucleotide may have the toxicity or immunogenicity; and some of which may lead to different conformations of the same base chain, so that for the same RNA sequence, there may be dozens of different conformation combinations, and this increases the difficulty of delivering it into the cell.

In July 2019, Professor Wei Wensheng's research group from Peking University firstly reports a nucleic acid editing technology: Leveraging Endogenous ADAR for Programmable Editing of RNA (LEAPER) in an article ⁴ published in Nature Biotechnology. Different from the CRISPR technology, on the one hand, this technology gets rid of the dependence on overexpression of the exogenous nucleases in principle, and may be completed by chemically synthesizing RNA, or may be delivered to the patient for fulfilling a function by vectors such as an adeno-associated virus (AAV) and a lentivirus, this makes the choice of its delivery measures more flexible and changeable, so that this technology has the greater advantage in the process of transforming to the medical field; and on the other hand, this technology may only achieve the editing from an adenosine A to a creatinine I (the creatinine I may be recognized as a guanosine G in the translation process), so that it is powerless for other mutations, for example, the T-to-C mutation. In addition, similar to the CRISPR technology, this technology also requires a section of RNA as a guide, to recruit the endogenous nuclease to a site that needs to be edited. This section of the guide RNA is named as an ADAR-recruiting RNA (arRNA).

In July 2019, Professor Zhang Feng's research group reports a new technology ¹ named as RNA Editing for Specific C to U Exchange (RESCUE). This technology is based on a basic skeleton of Cas13-ADAR reported by the research group in 2017, and a different mutation attempt is made on the catalytic domain of ADAR responsible for a reaction. Finally, the A-to-I editing activity of the ADAR catalytic domain is modified to the C-to-U editing activity, thereby the C-to-U editing on RNA in a specific site is achieved, and the precise editing range of the base is further expanded. However, this technology still requires the exogenous expression of Cas13 and the fusion protein after the ADAR mutation, and may not solve the problems caused by the bacteria-derived protein expression.

### SUMMARY OF THE INVENTION

In order to solve the problems in the above gene editing technologies, as to better apply the gene editing technologies to the medical field, it is urgent to find a targeted gene editing technology that is easy to deliver and may correct a T-to-C mutation with high efficiency and precision.

The present application creates a new RNA editing technology CUSPER, this technology does not depend on the expression of bacteria-derived Cas13b, and expands the application range of RNA editing from A-to-I editing to C-to-U editing.

In the present application, on the one hand, because it does not need to depend on the expression of a bacteria-derived macromolecular protein, a potential risk to an immune system and the impact on the editing efficiency of the system due to the attack of the immune system on a foreign protein are reduced; and on the other hand, with the significant reduction in the molecular weight of the protein that needs to be introduced, its delivery mode also becomes more flexible, and it may include chemical transformation and biological delivery, for example, AAV delivery, etc. Therefore, a technical scheme provided by the present application not only solves a technical problem of single-base editing from C to U, but also improves the safety, stability and use flexibility of an editing system, it is more beneficial to an in vivo application, and has an application prospect in the biomedicine field.

Specifically, the present application relates to:
1. An engineered composition or system for RNA editing, including:
1) a modified adenosine deaminase protein or a catalytic domain thereof or a construct expressing the modified adenosine deaminase protein or the catalytic domain thereof, wherein the adenosine deaminase protein or the catalytic domain thereof has the activity of catalyzing cytidine deamination after being modified, and
2) an arRNA that recruits the modified adenosine deaminase protein or the catalytic domain thereof to a target RNA, or a construct comprising the arRNA or a coding sequence for the arRNA;
   wherein the arRNA comprises a complementary RNA sequence that hybridizes to the target RNA, and the arRNA recruits the adenosine deaminase protein or the catalytic domain thereof to the target RNA, thereby deaminating a target cytidine in the target RNA.

In some embodiments, the construct expressing the modified adenosine deaminase protein or the catalytic domain thereof and the construct comprising the coding sequence for the arRNA are the same construct. In some embodiments, the construct expressing the modified adenosine deaminase protein or the catalytic domain thereof and the construct comprising the coding sequence for the arRNA are separated constructs.

In some embodiments, the modified adenosine deaminase protein of the present application is a protein that has the activity of cytidine deamination by deletion, addition or substitution of one or more amino acids in the adenosine deaminase protein (for example, an ADAR2 protein). In some embodiments, the modified adenosine deaminase protein of the present application is a protein that has the activity of cytidine deamination by substitution of one or more amino acids in the adenosine deaminase protein (for example, the ADAR2 protein) or the catalytic domain thereof. In some embodiments, the modified adenosine deaminase protein of the present application comprises the following mutation modifications: E488Q/V351G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1. In some embodiments, the modified adenosine deaminase protein of the present application comprises proteins with the corresponding mutations of E488Q/V351G/S486A/T375S/S370C/P462A/N597I/L332I/I398V/K350I/M383L/D619G/S58 2T/V440I/S495N/K418E/S661T using other ADAR2 proteins as reference sequences.

In some embodiments, the engineered composition or system for RNA editing of the present application comprises a catalytic domain of the above modified adenosine deaminase protein. In some embodiments, the catalytic domain is the catalytic domain of the above modified adenosine deaminase protein.

2. The engineered composition or system according to item 1, wherein the adenosine deaminase is modified by mutation in one or more sites to have the activity of deaminating cytidine which consequently converts to uridine.

3. The engineered composition or system according to item 2, wherein the adenosine deaminase protein or the catalytic domain thereof is an ADAR2 protein or a homologous protein or catalytic domain thereof.

4. The engineered composition or system according to item 3, wherein the mutation modification comprises: E488Q/V351G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1.

In some embodiments, the modified adenosine deaminase protein or the catalytic domain thereof is a homologous protein of the NP_001103.1 protein, and has a mutation corresponding to the following mutations: E488Q/V351G/S486A/T375S/S370C/P462A/N597I/L332I/I398V/K350I/M383L/D619G/S58 2T/V440I/S495N/K418E/S661T.

5. The engineered composition or system according to any one of items 1-4, wherein the arRNA hybridizes to the target RNA, and a targeting base in the arRNA opposite to the target cytidine is A, U, C, or G.

6. The engineered composition or system according to item 5, wherein the targeting base is U or C.

7. The engineered composition or system according to any one of items 1-6, wherein:
the arRNA comprises an unpaired nucleotide at one or more positions corresponding to upstream, downstream, or both upstream and downstream of a target base of the target RNA, to form a mismatch with the nucleotide at one or more positions of the upstream, downstream, or both upstream and downstream of the target base.

8. The engineered composition or system according to item 7, wherein the 3' most adjacent base of the arRNA targeting base forms a mismatch with the target RNA.

9. The engineered composition or system according to item 8, wherein the arRNA hybridizes to the target RNA, and the 3' most adjacent base of the targeting base forms a G-G mismatch with the target RNA.

10. The engineered composition or system according to any one of items 1-9, wherein the arRNA hybridizes to the target RNA, and the 5' most adjacent base of the targeting base does not form a mismatch with the target RNA.

11. The engineered composition or system according to item 10, wherein the 5' most adjacent base of the targeting base is U.

12. The engineered composition or system according to item 9, wherein the arRNA hybridizes to the target RNA, and an order of base preference from high to low in the target RNA opposite to the 5' most adjacent base of the targeting base of the arRNA is G or C, U or A(G≈C>U≈A).

13. The engineered composition or system according to any one of items 1-6, wherein the arRNA hybridizes to the target RNA, a target base triplet formed by the target base and the 5' and 3' adjacent bases thereof forms a mismatch only at the target base, wherein the target base triplet is selected from: ACG, ACC, UCC, UCG, CCC, CCG, UCA, and UCU.

14. The engineered composition or system according to any one of items 1-13, wherein the length of the arRNA is >50 nt, >55 nt, >60 nt, >65 nt, >70 nt, >75 nt, >80 nt, >85 nt, >90 nt, >95 nt, > 100 nt, > 105 nt, > 110 nt, > 115 nt, or > 120 nt. In some embodiments, the arRNA is about 151-53 nt, 131-61 nt, 121-61 nt, 111-65 nt, 101-71 nt, 91-71 nt, or 81-71 nt in length. In some specific embodiments, the length of the arRNA is any one positive integer within the length range defined by this item.

15. The engineered composition or system according to any one of items 1-14, wherein the lengths from the targeting base to the 3'-terminal and 5'-terminal in the arRNA are equal.

16. The engineered composition or system according to any one of items 1-14, wherein the length from the targeting base to the 3'-terminal in the arRNA is 45-5 nt, 40-5 nt, 35-10 nt, 25 nt-15 nt, or 24 nt-11 nt. In some specific embodiments, the length of the arRNA is any one positive integer within the length range defined by this item.

17. The engineered composition or system according to any one of items 1-14, wherein the length from the targeting base to the 5'-terminal in the arRNA is 80-30 nt, 70-35 nt, 60-40 nt, 55 nt-35 nt, or 55 nt -45 nt. In some specific embodiments, the length of the arRNA is any one positive integer within the length range defined by this item.

18. The engineered composition or system according to any one of items 1-17, wherein the arRNA is chemically modified.

19. The engineered composition or system according to item 18, wherein the chemical modification includes a 2'-O-methyl modification or an inter-nucleotide 3'-thio modification.

20. A method for deaminating a target cytosine in a target RNA in a cell, including introducing the following 1) and 2) into the cell:
1) a modified adenosine deaminase protein or a catalytic domain thereof or a construct expressing the modified adenosine deaminase protein or the catalytic domain thereof, and
2) an arRNA that recruits the modified adenosine deaminase protein or the catalytic domain thereof to the target RNA or a construct comprising the arRNA or a coding sequence for the arRNA, wherein the adenosine deaminase protein or the catalytic domain thereof has the activity of catalyzing cytidine deamination after being modified, and the arRNA comprises a complementary RNA sequence that hybridizes to the target RNA, and the arRNA recruits the adenosine deaminase protein or the catalytic domain thereof to the target RNA, thereby deaminating a target cytidine in the target RNA.

In some embodiments, the modified adenosine deaminase protein of the present application is a protein that has the activity of cytidine deamination by deletion, addition or substitution of one or more amino acids in the adenosine deaminase protein (for example, an ADAR2 protein). In some embodiments, the modified adenosine deaminase protein of the present application is a protein that has the activity of cytidine deamination by substitution of one or more amino acids in the adenosine deaminase protein (for example, the ADAR2 protein) or the catalytic domain thereof. In some embodiments, the modified adenosine deaminase protein of the present application comprises the following mutation modifications: E488Q/V351G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1. In some embodiments, the modified adenosine deaminase protein of the present application comprises proteins with the corresponding mutations of E488Q/V351G/S486A/T375S/S370C/P462A/N597I/L332I/I398V/K350I/M383L/D619G/S58 2T/V440I/S495N/K418E/S661T using other ADAR2 proteins as reference sequences.

In some embodiments, the engineered composition or system for RNA editing of the present application comprises a catalytic domain of the above modified adenosine deaminase protein.

21. The method according to item 20, wherein the construct expressing the modified adenosine deaminase protein or the catalytic domain thereof and the construct comprising the coding sequence for the arRNA are the same construct, and introduced into the cell simultaneously, or the construct expressing the modified adenosine deaminase protein or the catalytic domain thereof and the construct comprising the coding sequence for the arRNA are separated constructs, and the separated constructs are introduced into the cell simultaneously or separately.

22. The method according to item 20 or 21, wherein the adenosine deaminase is modified by mutation in one or more sites to have the activity of deaminating cytidine which subsequently converts to uridine.

23. The method according to item 22, wherein the adenosine deaminase protein or the catalytic domain thereof is an ADAR2 protein or a homologous protein or catalytic domain thereof.

24. The method according to item 23, wherein the mutation modification comprises: E488Q/V351G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1.

In some embodiments, the modified adenosine deaminase protein or the catalytic domain thereof is a homologous protein of the NP_001103.1 protein, and has a mutation corresponding to the following mutations: E488Q/V351G/S486A/T375S/S370C/P462A/N597I/L332I/I398V/K350I/M383L/D619G/S58 2T/V440I/S495N/K418E/S661T.

25. The method according to any one of items 21-24, wherein the arRNA hybridizes to the target RNA, and a targeting base in the arRNA opposite to the target cytidine is A, U, C, or G.

26. The method according to item 25, wherein the targeting base is U or C.

27. The method according to any one of items 21-26, wherein:
the arRNA comprises an unpaired nucleotide at one or more positions corresponding to upstream, downstream, or both upstream and downstream of a target base of the target RNA, to form a mismatch with the nucleotide at one or more positions of the upstream, downstream, or both upstream and downstream of the target base.

28. The method according to item 27, wherein the 3' most adjacent base of the arRNA targeting base forms a mismatch with the target RNA.

29. The method according to item 28, wherein the arRNA hybridizes to the target RNA, and the 3' most adjacent base of the targeting base forms a G-G mismatch with the target RNA.

30. The method according to any one of items 21-29, wherein the arRNA hybridizes to the target RNA, and the 5' most adjacent base of the targeting base does not form a mismatch with the target RNA.

31. The method according to item 30, wherein the 5' most adjacent base of the targeting base is U.

32. The method according to item 29, wherein the arRNA hybridizes to the target RNA, and an order of base preference from high to low in the target RNA opposite to the 5' most adjacent base of the targeting base of the arRNA is G or C, U or A (G≈C>U≈A).

33. The method according to any one of items 21-26, wherein the arRNA hybridizes to the target RNA, a target base triplet formed by the target base and the 5' and 3' adjacent bases thereof forms a mismatch only at the target base, wherein the target base triplet is selected from: ACG, ACC, UCC, UCG, CCC, CCG, UCA, and UCU.

34. The method according to any one of items 21-33, wherein the length of the arRNA is >50 nt, >55 nt, >60 nt, >65 nt, >70 nt, >75 nt, >80 nt, >85 nt, >90 nt, >95 nt, >100 nt, >105 nt, > 110 nt, > 115 nt, or > 120 nt. In some embodiments, the arRNA is about 151-53 nt, 131-61 nt, 121-61 nt, 111-65 nt, 101-71 nt, 91-71 nt, or 81-71 nt in length. In some specific embodiments, the length of the arRNA is any one positive integer within the length range defined by this item.

35. The method according to any one of items 21-34, wherein the lengths from the targeting base to the 3'-terminal and 5'-terminal in the arRNA are equal.

36. The method according to any one of items 21-34, wherein the length from the targeting base to the 3'-terminal in the arRNA is 45-5 nt, 40-5 nt, 35-10 nt, 25 nt-15 nt, or 24 nt-11 nt. In some specific embodiments, the length of the arRNA is any one positive integer within the length range defined by this item.

37. The method according to any one of items 21-34, wherein the length from the targeting base to the 5'-terminal in the arRNA is 80-30 nt, 70-35 nt, 60-40 nt, 55 nt-35 nt, or 55 nt -45 nt. In some specific embodiments, the length of the arRNA is any one positive integer within the length range defined by this item.

38. The method according to any one of items 21-37, wherein the arRNA is chemically modified.

39. The method according to item 38, wherein the chemical modification includes a 2'-O-methyl modification or an inter-nucleotide 3'-thio modification.

40. The method according to any one of items 21-39, wherein the cell is a mammalian cell.

41. A method for treating a disease caused by a T-to-C mutation, including using the method according to any one of items 21-39 to deaminate a target base C in a transcription-formed messenger RNA comprising the T-to-C mutation, to correct the mutation.

42. A modified adenosine deaminase protein, wherein the adenosine deaminase protein is ADAR2, it comprises mutation modifications of E488Q/V351G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1, and the ADAR2 protein has the activity of catalyzing cytidine deamination after being modified by the mutation.

In some embodiments, the modified adenosine deaminase protein or the catalytic domain thereof is a homologous protein of the NP_001103.1 protein, and has a mutation corresponding to the following mutations: E488Q/V351G/S486A/T375S/S370C/P462A/N597I/L332I/I398V/K350I/M383L/D619G/S58 2T/V440I/S495N/K418E/S661T.

43. A use of the modified adenosine deaminase protein according to item 42 for catalyzing cytidine deamination into a uridine.

### DESCRIPTION OF THE DRAWINGS

FIG. 1 shows a biologic false-positive (BFP) reporter system and a target base to be edited from C to U.
FIG. 2 shows a selection of the target base and a design principle of adjacent base at targeting sites while a guide RNA is designed by CUSPER and RESCUE technologies and a targeting point is U.
FIG. 3 shows a test of a CUSPER editing system. "/" in the figure represents that a corresponding plasmid or arRNA is not added, and only the same volume of water is added.
FIG. 4 shows a preference of LEAPER technology for the adjacent upstream and downstream bases of the target base. An arrow in the figure shows the preference of the LEAPER technology to 5' adjacent base while a 3' adjacent base of the target base corresponding to FIG. 3 is A.
FIG. 5 shows a preference of a RESCUE technology for the adjacent upstream and downstream bases of the target base. An arrow in the figure shows the preference of the RESCUE technology to the 5' adjacent base while the 3' adjacent base corresponding to FIG. 3 is A.
FIG. 6 shows a result of a repeated test on the CUSPER editing system. "/" in the figure represents that the corresponding plasmid or arRNA is not added, but only the same volume of water is added.
FIG. 7 shows sequences of mRNA and corresponding arRNA in the case that a target C corresponds to U, and its adjacent base does not have a mismatch.
FIG. 8 shows the C-to-U RNA editing efficiency for all 16 combinations of 3' and 5' adjacent bases in the case that the target C corresponds to U, and its adjacent base does not have the mismatch.

### DETAILED DESCRIPTION OF THE INVENTION

In order to solve the dilemma that existing gene editing technologies generally rely on heterologous foreign proteins and achieve the precise single-base editing on more types of bases, the present application creates an RNA editing technology CUSPER. CUSPER expands the application range of RNA editing from A-to-I editing to C-to-U editing, and because an enzyme protein expressed by a mammal itself is used for editing, it avoids the introduction of the heterologous foreign protein into a cell, thereby the safety is improved, and the entire gene editing process is more efficient and convenient.

### Definition

As used herein, the "CUSPER technology" is an original technology in a technical scheme of the present application, wherein CUSPER is the abbreviation of "C to U Specific Programmable Editing of RNA", namely "specific programmable RNA editing of converting a cytidine C into a uridine U". This technology uses a section of a short RNA that may complementarily hybridize to a target RNA, to recruit a modified adenosine deaminase protein or a protein comprising a catalytic domain thereof to the target RNA, as to deaminate a target cytidine, so that it is converted into a uridine. Wherein, the modified adenosine deaminase protein or the protein comprising the catalytic domain thereof has the activity of catalyzing cytosine deamination after being modified. The short RNA that may complementarily hybridize to the target RNA is arRNA. As used in the present application, "arRNA" refers to a single-stranded RNA that may recruit the adenosine deaminase protein or the catalytic domain thereof to the target RNA, and the arRNA recruits the adenosine deaminase protein or the catalytic domain thereof to the target RNA so that the target cytidine in the target RNA is deaminated. As used herein, "complementarity" of the nucleic acid refers to the ability of one nucleic acid to form a hydrogen bond with another nucleic acid by traditional Watson-Crick base pairing. Percentage complementarity represents a percentage of a residue in a nucleic acid molecule that may form the hydrogen bond (namely, Watson-Crick base pairing) with another nucleic acid molecule (for example, about 5, 6, 7, 8, 9, and 10 in 10 is about 50%, 60%, 70%, 80%, 90% and 100% of the complementary). "Complete complementarity" means that all continuous residues of a nucleic acid sequence form the hydrogen bond with the same number of the continuous residues in a second nucleic acid sequence. As used herein, "basic complementarity" refers to any one complementarity degree in at least about 70%, 75%, 80%, 85%, 90%, 95%, 97%, 98%, 99% or 100% in an area of about 40, 50, 60, 70, 80, 100, 150, 200, 250 or more nucleotides, or refers to two nucleic acids that hybridize under a stringent condition. For a single base or a single nucleotide, according to a Watson-Crick base pairing principle, while A is paired with T or U, and C is paired with G or I, it is called as complementarity or match, and vice versa; and other base pairings are all called as non-complementarity or mismatch.

"Hybridization" refers to a reaction in which one or more polynucleotides react to form a complex, and the complex is stabilized by a hydrogen bond between the bases of the nucleotide residues. The hydrogen bond may occur by Watson Crick base pairing, Hoogstein binding or any other sequence specific manners. A sequence capable of hybridizing to a given sequence is referred to as a "complementary sequence" of the given sequence.

As used herein, a term "delivery" refers to the introduction of biological macromolecules such as the nucleic acid and the protein from the outside of a cell membrane into the cell membrane by some ways. The "delivery" is, for example, electrotransfection, lipofection, lipid-nanoparticle delivery, virus delivery, and exosome delivery.

As used herein, a term "target RNA" refers to the target RNA to be edited, and it comprises the cytidine to be edited. The target RNA may be a mature mRNA or an mRNA precursor. The cytidine to be edited is referred to as "target base", "target cytidine" or "target C". The base adjacent to the target cytidine at the 5'-terminal of the target RNA is called as "5' adjacent base"; and the base adjacent to the target cytidine at the 3'-terminal of the target RNA is called as "3' adjacent base", and a base triplet formed by the target base and its 3' and 5' adjacent bases is referred to herein as a "target base triplet". While arRNA hybridizes to the target RNA, the base on the arRNA opposite to the target base is called as the "target base", the base adjacent to the target base at the 5'-terminal of the arRNA is called as the "5' most adjacent base", the base adjacent to the target base at the 3'-terminal of the arRNA is called as the "3' most adjacent base", and the base triplet formed by the target base and its 3' and 5' most adjacent bases is referred to herein as a "targeting base triplet".

In this article, the length of the targeting base from the 3'-termianl refers to the number of all bases from the 3' most adjacent base to the 3' most terminal base of the targeting base; and the length of the targeting base from the 5'-terminal refers to the number of all bases from the 5' most adjacent base to the 5' most terminal base of the targeting base.

As used herein, a term "ADAR" refers to a class of adenosine deaminase enzymes that are widely expressed in various tissues of eukaryotes (including mammals such as a human), and capable of catalyzing the conversion of an adenosine A to an inosine I in the RNA molecule.

In the present application, E488Q/V351G/S486A/T375S/S370C/P462A/N597I/L332I/I398V/K350I/M383L/D619G/S58 2T/V440I/S495N/K418E/S661T mutation refers to a series of mutations that occur in the ADAR2 protein, wherein the numbering of the amino acid of the mutation modification is consistent with the numbering of the amino acid in NP_001103.1, namely NP_001103.1 is used as a reference sequence. It should be known by those skilled in the art that the numbering of the amino acid in the mutation may be changed for the reference sequences of the different ADAR2 proteins. Therefore, as used in the present application, the E488Q/V351G/S486A/T375S/S370C/P462A/N597I/L332I/I398V/K350I/M383L/D619G/S58 2T/V440I/S495N/K418E/S661T mutation includes a mutation in the different ADAR2 protein reference sequences corresponding to a position of the mutated amino acid, the ADAR2 protein has the activity of catalyzing the cytidine deamination after being modified by the mutation. Correspondingly, the modified adenosine deaminase protein provided by the present application includes ADAR2 using NP_001103.1 as a reference sequence and comprising E488Q/V351G/S486A/T375S/S370C/P462A/N597I/L332I/I398V/K350I/M383L/D619G/S58 2T/V440I/S495N/K418E/S661T mutation modification, and also covers ADAR2 using different ADAR2 proteins as the reference sequences and comprising the corresponding mutation modification.

As used herein, a term "construct" refers to a nucleic acid vector comprising a certain nucleic acid sequence, and the nucleic acid vector may be a linear nucleic acid molecule, a plasmid or a virus vector, or the like. The nucleic acid molecule may be a single-stranded or double-stranded molecule. The specific nucleic acid sequence may be a DNA sequence or an RNA sequence. In some embodiments, the nucleic acid sequence functions directly without being transcribed, translated or expressed. In some embodiments, the nucleic acid sequence is the DNA sequence, and it functions in the form of the RNA molecule after being transcribed to form RNA. In some embodiments, the nucleic acid sequence is RNA, and it is translated to function in the form of a polypeptide or protein. In some embodiments, the nucleic acid sequence is DNA, and it functions in the form of a protein after transcription and translation steps to form the protein. The construct may enter the cell by packaging it into a virus, lipid nanoparticle or exosome, etc., or by electrotransformation, microinjection, chemical transformation and the like.

A term "modification" as used herein refers to changing the composition or structure of the nucleic acid or protein by chemical methods such as a genetic engineering method, thereby one or more properties or functions of the nucleic acid or protein are changed. For example, in the present application, the adenosine deaminase protein or the catalytic domain thereof has the effect of catalyzing the cytidine deamination after being modified, for example, addition, deletion and/or mutation of one or more amino acids.

### Engineered composition or system

The present application provides an engineered composition or system for RNA editing, including:
1) a modified adenosine deaminase protein or a catalytic domain thereof or a construct expressing the modified adenosine deaminase protein or the catalytic domain thereof, wherein the adenosine deaminase protein or the catalytic domain thereof has the activity of catalyzing cytidine deamination after being modified, and
2) an arRNA that recruits the modified adenosine deaminase protein or the catalytic domain thereof to a target RNA, or a construct comprising the arRNA or a coding sequence for the arRNA;
   wherein the arRNA comprises a complementary RNA sequence that hybridizes to the target RNA, and the arRNA recruits the adenosine deaminase protein or the catalytic domain thereof to the target RNA, thereby deaminating a target cytidine in the target RNA.

In some embodiments, the modified adenosine deaminase protein of the present application is a protein that has the activity of cytidine deamination by deletion, addition or substitution of one or more amino acids in the adenosine deaminase protein (for example, an ADAR2 protein). In some embodiments, the modified adenosine deaminase protein of the present application is a protein that has the activity of cytidine deamination by substitution of one or more amino acids in the adenosine deaminase protein (for example, the ADAR2 protein) or the catalytic domain thereof. In some embodiments, the modified adenosine deaminase protein of the present application comprises the following mutation modifications: E488Q/V351G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1. In some embodiments, the modified adenosine deaminase protein of the present application comprises proteins with the corresponding mutations of E488Q/V351G/S486A/T375S/S370C/P462A/N597I/L332I/I398V/K350I/M383L/D619G/S58 2T/V440I/S495N/K418E/S661T using other ADAR2 proteins as reference sequences.

In some embodiments, the engineered composition or system for RNA editing of the present application comprises the above modified adenosine deaminase protein or the catalytic domain thereof. In some embodiments, the catalytic domain is a catalytic domain of the above modified adenosine deaminase protein.

In some embodiments, the adenosine deaminase is modified by mutation in one or more sites to have the activity of cytidine deamination so that it is converted into the uridine. In some embodiments, the adenosine deaminase protein or the catalytic domain thereof is an ADAR2 protein or a homologous protein thereof or the catalytic domain of the ADAR2 protein or the catalytic domain of the ADAR2 protein homologous protein. In some embodiments, the mutation modification comprises: E488Q/V351G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1.

In some embodiments, the modified adenosine deaminase protein or the catalytic domain thereof is expressed by introducing a construct into a cell, and the construct is selected from any one of a linear nucleic acid, a plasmid, and a virus vector. In some embodiments, the cell is a eukaryotic cell. In some embodiments, the cell is a mammalian cell. In some embodiments, the cell is a human cell.

In some embodiments, while the arRNA hybridizes to the target RNA, its targeting base opposite to the target cytidine is A, U, C, or G. In some embodiments, the preferred targeting base order is U>C>A≈G, namely, while a plurality of arRNA sequences is compared, and in which all bases except the targeting base are the same, arRNA of which the targeting base is U or C generally has the higher editing efficiency. Thus preferably, in some embodiments, the targeting base is U or C.

In some embodiments, the arRNA comprises an unpaired nucleotide in one or more positions corresponding to upstream, downstream, or both upstream and downstream of the target base of the target RNA, to form the mismatch with the nucleotide in one or more positions of the upstream, downstream, or both upstream and downstream of the target base. In some embodiments, and the 3' most adjacent base of the arRNA targeting base forms the mismatch with the target RNA. In some embodiments, while the arRNA hybridizes to the target RNA, and the 3' most adjacent base of the targeting base forms a G-G mismatch with the target RNA. In some embodiments, while the arRNA hybridizes to the target RNA, the 5' most adjacent base of the targeting base does not form the mismatch with the target RNA. In some embodiments, the 5' most adjacent base of the targeting base does not form the mismatch with the target RNA, and wherein the 5' most adjacent base of the targeting base is U. In some embodiments, the arRNA hybridizes to the target RNA, and an order of base preference from high to low in the target RNA opposite to the 5' most adjacent base of the targeting base of the arRNA is G or C, U or A. In some embodiments, while the arRNA hybridizes complementarily to the target RNA, one or more bases except for the arRNA targeting triplet form the mismatch with the target RNA. In addition, in some embodiments, the mismatch may further improve the efficiency of targeted editing based on the arRNA.

In some embodiments, while the arRNA hybridizes to the target RNA, the target base triplet formed by the target base and its 5' and 3' adjacent bases forms the mismatch only at the target base, and, the lengths from the targeting base to the 3'-terminal and 5'-terminal in the arRNA are equal. At this time, preferably, the target base triplet is selected from: ACG, ACC, UCC, UCG, CCC, CCG, UCA, and UCU. In some embodiments, while the arRNA hybridizes to the target RNA, the target base triplet formed by the target base and its 5' and 3' adjacent bases forms the mismatch only at the target base, and, the lengths of the targeting base in the arRNA from the 3'-terminal and 5'-terminal are unequal. At this time, the target base triplet is selected from: ACG, ACC, UCC, UCG, CCC, CCG, UCA, and UCU.

In some embodiments, the length of the arRNA is >50 nt, >55 nt, >60 nt, >65 nt, >70 nt, >75 nt, >80 nt, >85 nt, >90 nt, >95 nt, > 100 nt, > 105 nt, > 110 nt, > 115 nt, or > 120 nt. In some embodiments, the arRNA is about 151-53 nt, 131-61 nt, 121-61 nt, 111-65 nt, 101-71 nt, 91-71 nt, or 81-71 nt in length. In some specific embodiments, the length of the arRNA is any one positive integer within the length range defined by this item.

In some embodiments, the lengths from the targeting base to the 3'-terminal and 5'-terminal in the arRNA are equal. In some embodiments, the lengths of the targeting base in the arRNA from the 3'-terminal and 5'-terminal are unequal. In some embodiments, the length from the targeting base to the 3'-terminal in the arRNA is 45-5 nt, 40-5 nt, 35-10 nt, 25 nt-15 nt, or 24 nt-11 nt. In some specific embodiments, the length is selected from any one positive integer within the length range defined by this item.

In some embodiments, the length from the targeting base to the 5'-terminal in the arRNA is 80-30 nt, 70-35 nt, 60-40 nt, 55 nt-35 nt, or 55 nt-45 nt. In some specific embodiments, the length is selected from any one positive integer within the length range defined by this item. In some embodiments, the length from the targeting base to the 5'-terminal in the arRNA is greater than 80.

In some embodiments, the arRNA is chemically synthesized. In some embodiments, the arRNA is an oligonucleotide. In some embodiments, the arRNA is chemically modified. In some embodiments, the chemical modification includes a 2'-O-methyl modification or an inter-nucleotide 3'-thio modification. In some embodiments, the chemical modification is selected from one or more of the followings:
the first 3 nucleotides and the last 3 nucleotides of the sequence are modified by 2'-OMe respectively,
the first 3 and the last 3 inter-nucleotide linkages are phosphorothioate bond linkages,
all U in the sequence are modified by 2'-OMe,
the 3' most adjacent base of the targeting base is A modified by 2'-OMe,
the 5' most adjacent base of the targeting base is C modified by 2'-OMe,
the targeting base is linked to its 3' most adjacent base and 5' most adjacent base by phosphorothioate bonds respectively,
the first 5 and last 5 nucleotides are modified by 2'-OMe respectively, and
the first 5 and last 5 inter-nucleotide linkages are the phosphorothioate bond linkages.

In some embodiments, the arRNA is encoded by a construct and generated by transcription. In some embodiments, the construct is selected from a linear nucleic acid chain, a virus vector or a plasmid.

### Method for editing RNA

The present application provides a method for deaminating a target cytosine in a target RNA in a cell, CUSPER (programmed C to U RNA editing), including introducing the following 1) and 2) into the cell:
1) a modified adenosine deaminase protein or a catalytic domain thereof or a construct expressing the modified adenosine deaminase protein or the catalytic domain thereof, and
2) an arRNA that recruits the modified adenosine deaminase protein or the catalytic domain thereof to a target RNA, or a construct comprising the arRNA or a coding sequence for the arRNA; wherein the adenosine deaminase protein or the catalytic domain thereof has the activity of catalyzing cytidine deamination after being modified, and the arRNA comprises a complementary RNA sequence that hybridizes to the target RNA, and the arRNA recruits the adenosine deaminase protein or the catalytic domain thereof to the target RNA, thereby deaminating a target cytidine in the target RNA.

In some embodiments, the cell is a eukaryotic cell. In some embodiments, the cell is a mammalian cell. In some embodiments, the cell is a human cell. In some embodiments, the cell is a mouse cell.

In some embodiments, the modified adenosine deaminase protein of the present application is a protein that has the activity of cytidine deamination by deletion, addition or substitution of one or more amino acids in the adenosine deaminase protein (for example, an ADAR2 protein). In some embodiments, the modified adenosine deaminase protein of the present application is a protein that has the activity of cytidine deamination by substitution of one or more amino acids in the adenosine deaminase protein (for example, the ADAR2 protein) or the catalytic domain thereof. In some embodiments, the modified adenosine deaminase protein of the present application comprises the following mutation modifications: E488Q/V351G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1. In some embodiments, the modified adenosine deaminase protein of the present application comprises proteins with the corresponding mutations of E488Q/V351G/S486A/T375S/S370C/P462A/N597I/L332I/I398V/K350I/M383L/D619G/S58 2T/V440I/S495N/K418E/S661T using other ADAR2 proteins as reference sequences.

It should be known by those skilled in the art that the construct expressing the modified adenosine deaminase protein or the catalytic domain thereof comprises the coding sequence of the modified adenosine deaminase protein or the catalytic domain thereof. In some embodiments, the construct expressing the modified adenosine deaminase protein or the catalytic domain thereof and the construct comprising the coding sequence for the arRNA are the same construct. In some embodiments, the construct expressing the coding sequence of the modified adenosine deaminase protein or the catalytic domain thereof and the construct comprising the coding sequence for the arRNA are the different constructs, namely the coding sequence of the modified adenosine deaminase protein or the catalytic domain thereof and the coding sequence for the arRNA are respectively located on the different constructs. In some embodiments, the different constructs are two or more than two constructs. In some embodiments, the different constructs are introduced into the cell simultaneously or separately. It should be understood that the different constructs mentioned in this paragraph refer to the non-same construct, and it does not mean that the different constructs belong to different categories of the constructs respectively. In some embodiments, the construct expressing the modified adenosine deaminase protein or the catalytic domain thereof and the arRNA that recruits the modified adenosine deaminase protein or the catalytic domain thereof to the target RNA or the construct comprising the arRNA or the construct comprising the coding sequence for the arRNA are introduced into the same cell. In some embodiments, the modified adenosine deaminase protein or the catalytic domain thereof or the construct expressing the modified adenosine deaminase protein or the catalytic domain thereof, and a plurality of arRNAs or the construct comprising the arRNA or the construct comprising the coding sequence for the arRNA are introduced into the cell, to achieve high-throughput editing of the target RNA, wherein the plurality of the arRNAs is arRNAs targeting the different target RNAs, or arRNAs targeting the different target base (for example, C) sites in the same target RNA.

Therefore, the present application also covers a method for high-throughput editing of a target cytosine in a target RNA in a cell, including introducing the following 1) and 2) into the cell:
1) a modified adenosine deaminase protein or a catalytic domain thereof or a construct expressing the modified adenosine deaminase protein or the catalytic domain thereof, and
   the modified adenosine deaminase protein or the catalytic domain thereof are recruited to a plurality (for example, 2 or more, 5 or more, 5 or more, and 10 or more) of arRNAs of the target RNA or the construct comprising the arRNA or comprising its coding sequence, wherein the adenosine deaminase protein or the catalytic domain thereof has the activity of catalyzing cytidine deamination after being modified, the plurality (for example, 2 or more, 5 or more, 5 or more, and 10 or more) of the arRNAs comprises a complementary RNA sequence that hybridizes to the target RNA respectively, and the arRNA recruits the adenosine deaminase protein or the catalytic domain thereof to the target RNA so that the target cytidine is the target RNA is deaminated.

In some embodiments, the construct expressing the modified adenosine deaminase protein or the catalytic domain thereof and the construct comprising the coding sequence for the arRNA are the same construct, or the construct expressing the modified adenosine deaminase protein or the catalytic domain thereof and the construct comprising the coding sequence for the arRNA are separated constructs, and the separated constructs are introduced into the cell simultaneously or separately.

In some embodiments, the adenosine deaminase is modified by mutation in one or more sites to have the activity of deaminating cytidine which consequently converts to uridine.

In some embodiments, the adenosine deaminase protein or the catalytic domain thereof is an ADAR2 protein or a homologous protein or catalytic domain thereof.

In some embodiments, the mutation modification comprises: E488Q/V351G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1. In some embodiments, the construct is selected from a virus vector, a plasmid and a linear nucleic acid. In some embodiments, the modified adenosine deaminase protein or the catalytic domain thereof or the construct expressing the modified adenosine deaminase protein or the catalytic domain thereof and the construct comprising the oligonucleotide that recruits the modified adenosine deaminase protein or the catalytic domain thereof to the arRNA of the target RNA or transcribing the arRNA are introduced into the cell by modes such as virus infection, chemical transfection, electrotransfection, exosome delivery, or nano-lipid particle delivery.

In some embodiments, the adenosine deaminase is modified by mutation in one or more sites to have the activity of deaminating the cytidine so that it is converted in the uridine. In some embodiments, the adenosine deaminase protein or the catalytic domain thereof is the ADAR2 protein or the homologous protein or catalytic domain thereof. In some embodiments, the mutation modification comprises: E488Q/V351G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1.

In some embodiments, while the arRNA hybridizes to the target RNA, its targeting base opposite to the target cytidine is A, U, C, or G. In some embodiments, while the arRNA hybridizes to the target RNA, its targeting base opposite to the target cytidine is preferably U or C.

In some embodiments, the arRNA comprises an unpaired nucleotide in one or more positions corresponding to upstream, downstream, or both upstream and downstream of the target base of the target RNA, to form the mismatch with the nucleotide in one or more positions of the upstream, downstream, or both upstream and downstream of the target base. In some embodiments, and the 3' most adjacent base of the arRNA targeting base forms the mismatch with the target RNA. In some embodiments, while the arRNA hybridizes to the target RNA, and the 3' most adjacent base of the targeting base forms a G-G mismatch with the target RNA. In some embodiments, while the arRNA hybridizes to the target RNA, the 5' most adjacent base of the targeting base does not form the mismatch with the target RNA. In some embodiments, the 5' most adjacent base of the targeting base is U. In some embodiments, the arRNA hybridizes to the target RNA, and an order of base preference from high to low in the target RNA opposite to the 5' most adjacent base of the targeting base of the arRNA is G or C, U or A (G≈C>U≈A) from high to low. In some embodiments, while the arRNA hybridizes to the target RNA, the base in the target RNA opposite to the 5' most adjacent base of the targeting base of the arRNA is preferably G or C. In some embodiments, while the arRNA hybridizes to the target RNA, the base in the target RNA opposite to the 5' most adjacent base of the targeting base of the arRNA is most preferably G. In some embodiments, while the arRNA hybridizes complementarily to the target RNA, one or more bases except for the arRNA targeting triplet form the mismatch with the target RNA. In addition, in some embodiments, the mismatch may further improve the efficiency of targeted editing based on the arRNA.

In some embodiments, while the arRNA hybridizes to the target RNA, the target base triplet formed by the target base and its 5' and 3' adjacent bases forms the mismatch only at the target base, and, the lengths from the targeting base to the 3'-terminal and 5'-terminal in the arRNA are equal. At this time, preferably, the target base triplet is selected from: ACG, ACC, UCC, UCG, CCC, CCG, UCA, and UCU. In some embodiments, while the arRNA hybridizes to the target RNA, the target base triplet formed by the target base and its 5' and 3' adjacent bases forms the mismatch only at the target base, and, the lengths of the targeting base in the arRNA from the 3'-terminal and 5'-terminal are unequal. At this time, the target base triplet is selected from: ACG, ACC, UCC, UCG, CCC, CCG, UCA, and UCU.

In some embodiments, the length of the arRNA is >50 nt, >55 nt, >60 nt, >65 nt, >70 nt, >75 nt, >80 nt, >85 nt, >90 nt, >95 nt, > 100 nt, > 105 nt, > 110 nt, > 115 nt, or > 120 nt. In some embodiments, the arRNA is about 151-53 nt, 131-61 nt, 121-61 nt, 111-65 nt, 101-71 nt, 91-71 nt, or 81-71 nt in length. In some specific embodiments, the length of the arRNA is any one positive integer within the length range defined by this item.

In some embodiments, the lengths from the targeting base to the 3'-terminal and 5'-terminal in the arRNA are equal. In some embodiments, the lengths of the targeting base in the arRNA from the 3'-terminal and 5'-terminal are unequal. In some embodiments, the length from the targeting base to the 3'-terminal in the arRNA is 45-5 nt, 40-5 nt, 35-10 nt, 25 nt-15 nt, or 24 nt-11 nt. In some specific embodiments, the length is selected from any one positive integer within the length range defined by this item.

In some embodiments, the length from the targeting base to the 5'-terminal in the arRNA is 80-30 nt, 70-35 nt, 60-40 nt, 55 nt-35 nt, or 55 nt-45 nt. In some specific embodiments, the length is selected from any one positive integer within the length range defined by this item. In some embodiments, the length from the targeting base to the 5'-terminal in the arRNA is greater than 80.

In some embodiments, the arRNA is an oligonucleotide or contained in the oligonucleotide. In some embodiments, the oligonucleotide is chemically modified. In some embodiments, the chemical modification includes a 2'-O-methyl modification or an inter-nucleotide 3'-thio modification. In some embodiments, the chemical modification is selected from one or more of the followings:
the first 3 nucleotides and the last 3 nucleotides of the sequence are modified by 2'-OMe respectively,
the first 3 and the last 3 inter-nucleotide linkages are phosphorothioate bond linkages,
all U in the sequence are modified by 2'-OMe,
the 3' most adjacent base of the targeting base is A modified by 2'-OMe,
the 5' most adjacent base of the targeting base is C modified by 2'-OMe,
the targeting base is linked to its 3' most adjacent base and 5' most adjacent base by phosphorothioate bonds respectively,
the first 5 and last 5 nucleotides are modified by 2'-OMe respectively, and
the first 5 and last 5 inter-nucleotide linkages are the phosphorothioate bond linkages.

In some embodiments, the arRNA is encoded by a construct and generated by transcription. In some embodiments, the construct is selected from a linear nucleic acid chain, a virus vector or a plasmid.

### RNA editing related enzyme protein and use thereof

The present application provides a modified adenosine deaminase protein, wherein the adenosine deaminase protein is ADAR2, and it comprises the following amino acid mutations corresponding to ADAR2 of Genebank accession number NP_001103.1: E488Q/V351G/S486A/T375S/S370C/P462A/N597I/L332I/I398V/K350I/M383L/D619G/S58 2T/V440I/S495N/K418E/S661T, or an amino acid mutation in the corresponding position of a homologous ADAR2 protein of the NP_001103.1, and the ADAR2 protein has the activity of catalyzing cytidine deamination after being modified by the mutation.

The present application further provides a use of the modified adenosine deaminase protein for catalyzing deamination of a cytidine into a uridine. In some embodiments, the use of catalyzing the deamination of the cytidine into the uridine occurs intracellularly. In some embodiments, the use of catalyzing the deamination of the cytidine into the uridine occurs extracellularly.

### Disease therapeutic method

The present application provides a method for treating a disease caused by a T-to-C mutation, including using the above method for editing RNA to deaminate a target base C in a transcription-formed messenger RNA comprising the T-to-C mutation, to correct the mutation.

In some embodiments, the method for treating the disease caused by the T-to-C mutation includes injecting the above engineered composition or system into a subject. In some embodiments, the treatment may include injecting the following 1) and 2) into the subject.
1) A modified adenosine deaminase protein or a catalytic domain thereof or a construct expressing the modified adenosine deaminase protein or the catalytic domain thereof, and
2) an arRNA that recruits the modified adenosine deaminase protein or the catalytic domain thereof to the target RNA or a construct comprising the arRNA or a coding sequence for the arRNA, wherein, the adenosine deaminase protein is the above RNA editing related enzyme protein.

In some embodiments, the injection is intravenous injection, arterial infusion, intramuscular injection, subcutaneous injection, or intratumoral injection.

In some embodiments, the diseases caused by the T-to-C mutation include genetic diseases and cancers.

### Kit and preparation

The present application further provides a kit, and it is used for catalyzing deamination of a cytidine into a uridine. In some embodiments, the kit comprises the above engineered composition or system. In some embodiments, the kit comprises a construct encoding or expressing the modified adenosine deaminase protein or the catalytic domain thereof in the engineered composition system and/or recruiting the modified adenosine deaminase protein or the catalytic domain thereof to arRNA of the target RNA.

The method for targeted editing of RNA using the CUSPER technology provided by the present application has the following advantages:
On the one hand, while the new technology recruits the editing protein (such as ADAR), unlike the RESCUE technology, it is necessary to design the guide RNA comprising a Cas13b recruiting framework, and it does not require the overexpression of bacteria-derived Cas13b, the length of the exogenous expressed protein is reduced so that it is easy and diverse to be loaded by the virus vector and delivered in the human body, and at the same time, it may also reduce the possibility of gene editing failure caused by the neutralization of the exogenously expressed nuclease, and this makes it a significant advantage over the RESCUE technology while applied to the medical field.

On the other hand, the new system is not like the LEAPER technology, the LEAPER technology may only achieve the editing of the RNA editing application ranging from A to I, while the new system widens the editing to C-to-U, so that many genetic diseases that show the T-to-C mutation on the genomes, and other applications that require the conversion of C to T/U may treated with the technology of the present application.

Therefore, compared with the prior art, the technology of the present application has a wider application range, and is safer and more effective.

Preferred embodiments of the present invention are described above in detail, however, the present invention is not limited this. Within a scope of the technical concept of the present invention, a plurality of simple modifications may be made to the technical solutions of the present invention, including combining various technical features in any other suitable manners. These simple modifications and combinations should also be regarded as the content disclosed in the present invention, and all belong to a scope of protection of the present invention.

Hereinafter, the technical solutions of the present invention are further described in detail below with reference to specific embodiments, but the present invention is not limited to the following examples. Unless otherwise specified, the reagents mentioned below are all commercially available. For the sake of brevity, some operations do not detail operation parameters, steps and used instruments, and it should be understood that these are well-known and reproducible by those skilled in the art.

### Example

### Example 1: molecular construction of modified ADAR2 and BFP reporter system

### 1. Construction of mutant ADAR2-r16-293T

The catalytic domain of RNA adenosine deaminase 2 (ADAR2) is mutagenized with reference to the RESCUE technology reported in a reference document 1, a mutation site is the same as r16 in the document (dADAR2(E488Q/V351G/S486A/T375S/S370C/P462A/N597I/L332I/I398V/K350I/M383L/ D619G/S582T/V440I/S495N/K418E/S661T) r16, https://benchling.com/s/seq-19Ytwwh0i0vSIbyXYZ95), wherein the numbering of the amino acid of the mutation modification is consistent with the numbering of the amino acid in NP_001103.1. An ADAR2 coding sequence fragment comprising the above mutation is synthesized in vitro by using a conventional DNA synthesis technology in the field, and inserted between an ADAR2 XmaI restriction site and an AscI restriction site of a pLenti-ADAR2-r16 plasmid vector (a pLenti-ADAR2 plasmid backbone is donated by Professor Wei Wensheng' laboratory) by restriction enzyme ligation. The plasmid constructed by the above steps is named pLenti-ADAR2-r16, and an ADAR2 gene thereof comprising the above mutation is named ADAR2-r16. The full-length cDNA sequence of ADAR2-r16 is: SEQ ID NO 1. By a second generation lentiviral packaging system (pCAG-VSVG is donated by Arthur Nienhuis & Patrick Salmon (Addgene plasmid #35616; http://n2t.net/addgene:35616; RRID:Addgene_35616); pCMVR8.74 is donated by Didier Trono (Addgene plasmid # 22036; http://n2t.net/addgene:22036; RRID:Addgene_22036)), pLenti-ADAR2-r16 is packaged into a lentivirus, and the lentivirus is used to infect 293T cells and resistance screening is performed by using Blasticidin (Solarbio B9300) with a final concentration of 10 ug/ml after 48 hours. After the resistance screening, the survived cells are called as ADAR2-r16-293T.

SEQ ID NO 1:
ATGGATATAGAAGATGAAGAAAACATGAGTTCCAGCAGCACTGATGTGAA
GGAAAACCGCAATCTGGACAACGTGTCCCCCAAGGATGGCAGCACACCTGGGCC
TGGCGAGGGCTCTCAGCTCTCCAATGGGGGTGGTGGCCCCGGCAGAAAGCG
GCCCCTGGAGGAGGGCAGCAATGGCCACTCCAAGTACCGCCTGAAGAAAAGGAG
GAAAACACCAGGGCCCGTCCTCCCCAAGAACGCCCTGATGCAGCTGAATGAGAT
CAAGCCTGGTTTGCAGTACACACTCCTGTCCCAGACTGGGCCCGTGCACGCGCCT
TTGTTTGTCATGTCTGTGGAGGTGAATGGCCAGGTTTTTGAGGGCTCTGGTCCCAC
AAAGAAAAAGGCAAAACTCCATGCTGCTGAGAAGGCCTTGAGGTCTTTCGTTCAG
TTTCCTAATGCCTCTGAGGCCCACCTGGCCATGGGGAGGACCCTGTCTGTCAACA
CGGACTTCACATCTGACCAGGCCGACTTCCCTGACACGCTCTTCAATGGTTTTGAA
ACTCCTGACAAGGCGGAGCCTCCCTTTTACGTGGGCTCCAATGGGGATGACTCCT
TCAGTTCCAGCGGGGACCTCAGCTTGTCTGCTTCCCCGGTGCCTGCCAGCCTAGCC
CAGCCTCCTCTCCCTGCCTTACCACCATTCCCACCCCCGAGTGGGAAGAATCCCGT
GATGATCTTGAACGAACTGCGCCCAGGACTCAAGTATGACTTCCTCTCCGAGAGC
GGGGAGAGCCATGCCAAGAGCTTCGTCATGTCTGTGGTCGTGGATGGTCAGTTCT
TTGAAGGCTCGGGGAGAAACAAGAAGCTTGCCAAGGCCCGGGCTGCGCAGTCTG
CCCTGGCCGCCATTTTTAACTTGCACTTGGATCAGACGCCATCTCGCCAGCCTATT
CCCAGTGAGGGTCTTCAGCTGCATTTACCGCAGGTTTTAGCTGACGCTGTCTCACG
CCTGGTCATAGGTAAGTTTGGTGACCTGACCGACAACTTCTCCTCCCCTCACGCTC
GCAGAATAGGTCTGGCTGGAGTCGTCATGACAACAGGCACAGATGTTAAAGATG
CCAAGGTGATATGTGTTTCTACAGGATCTAAATGTATTAATGGTGAATACCTAAG
TGATCGTGGCCTTGCATTAAATGACTGCCATGCAGAAATAGTATCTCGGAGATCC
TTGCTCAGATTTCTTTATACACAACTTGAGCTTTACTTAAATAACGAGGATGATCA
AAAAAGATCCATCTTTCAGAAATCAGAGCGAGGGGGGTTTAGGCTGAAGGAGAA
TATACAGTTTCATCTGTACATCAGCACCTCTCCCTGTGGAGATGCCAGAATCTTCT

CACCACATGAGGCAATCCTGGAAGAACCAGCAGATAGACACCCAAATCGTAAAG
CAAGAGGACAGCTACGGACCAAAATAGAGGCTGGTCAGGGACGATTCCAGTGC
GCAACAATGCGAGCATCCAAACGTGGACGGGGTGCTGCAAGGGAGCGGCTGC
TCACCATGTCCTGCAGTGACAAGATTGCACGCTGGAACGTGGTGGGCATCCAGGG
ATCACTGCTCAGCATTTTCGTGGAGCCCATTTACTTCTCGAGCATCATCCTGGGCA
GCCTTTACCACGGGGACCACCTTTCCAGGGCCATGTACCAGCGGATCTCCAACAT
AGAGGACCTGCCACCTCTCTACACCCTCAACAAGCCTTTGCTCACAGGCATCAGC
AATGCAGAAGCACGGCAGCCAGGGAAGGCCCCCATATTCAGTGTCAACTGGACG
GTAGGCGACTCCGCTATTGAGGTCATCAACGCCACGACTGGGAAGGGAGAGCTG
GGCCGCGCGTCCCGCCTGTGTAAGCACGCGTTGTACTGTCGCTGGATGCGTGTGC
ACGGCAAGGTTCCCTCCCACTTACTACGCTCCAAGATTACCAAGCCCAACGTGTA
CCATGAGACAAAGCTGGCGGCAAAGGAGTACCAGGCCGCCAAGGCGCGTCTGTT
CACAGCCTTCATCAAGGCGGGGCTGGGGGCCTGGGTGGAGAAGCCCACCGAGCA
GGACCAGTTCTCACTCACGCCCGATTACAAGGATGACGACGATAAGTAG

### 2. Construction of BFP reporter system

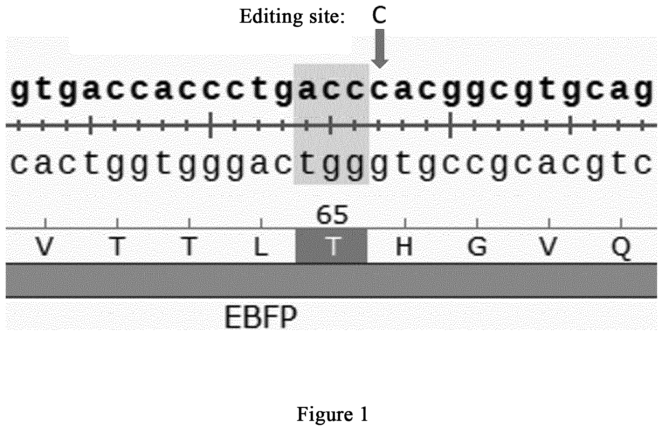

The BFP reporter system is constructed with reference to a reference document 7, the entire BFP (blue fluorescent protein) cDNA sequence is synthesized in vitro, and the specific sequence is: SEQ ID NO 2. The BFP cDNA sequence is cloned into the pCDH-CMV plasmid vector by a multiple cloning site behind a CMV promoter (a pCDH-CMV plasmid backbone is donated by Kazuhiro Oka, Addgene plasmid # 72265; http://n2t.net/addgene:72265; RRID: Addgene_72265). The target base to be edited in the reporter system is a base C at the 199th position of the BFP sequence, corresponding to histidine at the position 66th, and referring to FIG. 1.

SEQ ID NO 2:
ATGGTGAGCAAGGGCGAGGAGCTGTTCACCGGGGTGGTGCCCATCCTGGTCGAGCTG
GACGGCGACGTAAACGGCCACAAGTTCAGCGTGTCTGGCGAGGGCGAGGGCGATGCCACCTA
CGGCAAGCTGACCCTGAAGTTCATCTGCACCACCGGCAAGCTGCCCGTGCCCTGGCCCACCCT
CGTGACCACCCTGACCCACGGCGTGCAGTGCTTCAGCCGCTACCCCGACCACATGAAGCAGC
ACGACTTCTTCAAGTCCGCCATGCCCGAAGGCTACGTCCAGGAGCGCACCATCTTCTTCAAGG
ACGACGGCAACTACAAGACCCGCGCCGAGGTGAAGTTCGAGGGCGACACCCTGGTGAACCGC

ATCGAGCTGAAGGGCATCGACTTCAAGGAGGACGGCAACATCCTGGGGCACAAGCTGGAGTA
CAACTACAACAGCCACAACGTCTATATCATGGCCGACAAGCAGAAGAACGGCATCAAGGCGA
ACTTCAAGATCCGCCACAACATCGAGGACGGCAGCGTGCAGCTCGCCGACCACTACCAGCAG
AACACCCCCATCGGCGACGGCCCCGTGCTGCTGCCCGACAACCACTACCTGAGCACCCAGTCC
GCCCTGAGCAAAGACCCCAACGAGAAGCGCGATCACATGGTCCTGCTGGAGTTCGTGACCGC
CGCCGGGATCACTCTCGGCATGGACGAGCTGTACAAGTGA

Bases at the 198th, 199th, and 200th positions of the sequence are CCA successively, named BFP-CCA, and abbreviated as C^{∗}. After the base C at the 199th position is edited to U by deamination at the RNA level, the BFP fluorescent protein may change from an original blue fluorescence to a green fluorescence, thereby a signal may be detected by a flow cytometry fluorescein isothiocyanate (FITC) channel. Since the nucleotide at the 198th position is mutated from C to A, T, and G, the corresponding codons (bases at 196th, 197th, and 198th positions) ACC, ACA, ACT, and ACG all encode a threonine, so the mutation at this site is a synonymous mutation. This allows the reporter system to simultaneously measure and compare the editing efficiency of C to U while the 5' upstream adjacent bases of the target base on the mRNA are different. Based on this, a site-directed mutagenesis kit (Q5^{®} Site-Directed Mutagenesis Kit, NEB E0554S) may be used to introduce the mutation into the base at the 198th position, so that the bases at the 198th, 199th, and 200th positions are respectively: GCA, named BFP-GCA, and abbreviated as G^{∗}; ACA, named BFP-ACA, and abbreviated as A^{∗}; and TCA, named BFP-UCA, and abbreviated as U^{∗}.

### Example 2: Preliminary test of CUSPER system

### 1. Design and synthesis of arRNA

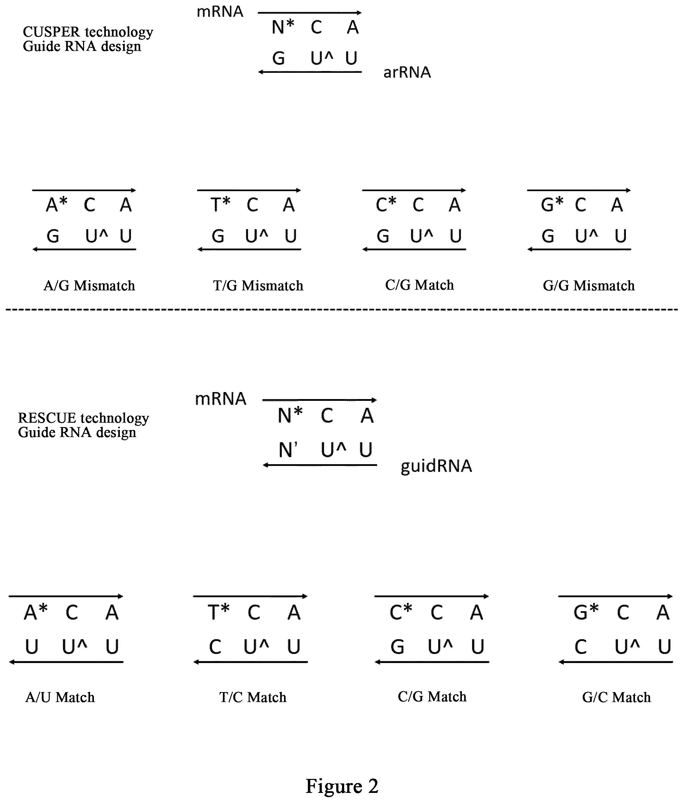

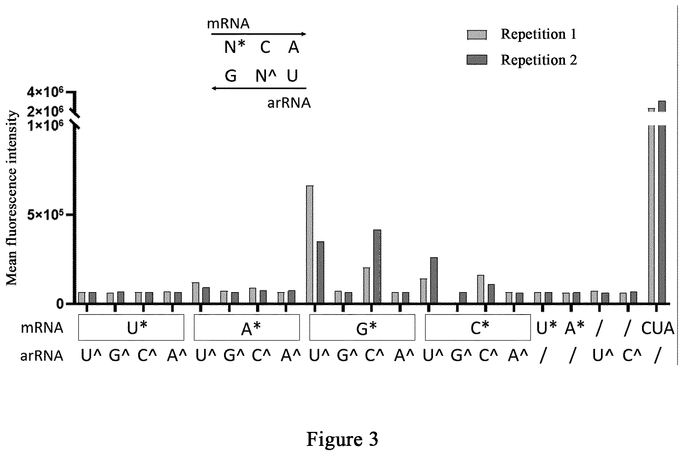

The arRNA used in this example comprises an antisense RNA complementary to the target mRNA, the target base is located in a middle position of the arRNA, and the 5' upstream and 3' downstream extend to both sides in the same length. Due to the limitation of the synthesis length, in this example, RNA with a length of 91 nt is selected for in vitro synthesis. As shown in FIG. 3, while the nucleotide at position 46 of the arRNA is A, U, G, and C respectively, it is abbreviated as A^, U^, G^, and C^ respectively. The specific sequences of the four synthesized arRNAs are shown in Table 1 below. As shown in FIG. 2, different from the LEAPER technology design method, the design of the four arRNAs in this batch of experiments, because the purpose of the experiment is to determine the editing efficiency while the target base is mismatched with the targeting base on the arRNA, uses the different targeting bases for testing, namely the arRNAs with A, U, G, and C at 46th position are tested. The 5' most adjacent base at the 47th position of the arRNA (corresponding to the 198th position of the reporter system) is designed according to the target triplet base (namely CCA) of the BFP sequence before the introduction of the mutation, namely the 5' most adjacent base (the 47th position) of the targeting base of the arRNA is U complementary to A. This means that the design of the arRNA is consistent with the LEAPER technology if and only if while the reporter system of the subsequent test is BFP-CCA, namely: while the arRNA hybridizes to the target RNA, there is a mismatch only at the target base; and while the reporter system is BFP-GCA, BFP-TCA, BFP-ACA, the design of the arRNA not only has the mismatch at the target base, but also has the mismatch at the 3' adjacent base of the arRNA target base.

**Table 1**

| arRNA name | arRNA sequence | | | |
|---|---|---|---|---|
| A^ | SEQ | ID | NO | 3: |
| | gcugcuucauguggucggguagcggcugaagcacugcacgccguAggucaggguggucacgaggg ugggccagggcacgggcagcuugcc | | | |
| U^ | SEQ | ID | NO | 4: |
| | gcugcuucauguggucggguagcggcugaagcacugcacgccguUggucaggguggucacgaggg ugggccagggcacgggcagcuugcc | | | |
| C^ | SEQ | ID | NO | 5: |
| | gcugcuucauguggucggguagcggcugaagcacugcacgccguCggucaggguggucacgaggg ugggccagggcacgggcagcuugcc | | | |
| G^ | SEQ | ID | NO | 6: |
| | gcugcuucauguggucggguagcggcugaagcacugcacgccguGggucaggguggucacgaggg ugggccagggcacgggcagcuugcc | | | |

| | | | | |
|---|---|---|---|---|
| Note: Capital letters are only used to highlight differences between the sequences, and the different capital and small letters of the same letter does not represent the differences in the base. | | | | |

### 2. C-to-U editing test

ADAR2-r16-293T is plated to a 6-well plate at a density of 300,000 cells/well, and 24 hours after plating, it is transfected with Lipofectamine 3000 (Invitrogen L3000015), and transfection steps are performed according to the instruction. According to the instruction, the different concentrations of Lipofectamine 3000 transfection reagents are used to perform two times of the repeated experiment. Repetition 1: 3.75 µl of Lipofectamine 3000 is used per well, and repetition 2: 7.5 µl of Lipofectamine 3000 is used per well. 2.5 µg of the BFP reporter plasmid (selected from: BFP-GCA, abbreviated as G^{∗}; BFP-ACA, abbreviated as A^{∗}; BFP-TCA, abbreviated as T^{∗}; and BFP-CCA, abbreviated as C^{∗}) and 25 pmol of chemically synthesized arRNA are added per well. The FITC channel signal intensity is detected by a fluorescence activated cell sorter (FACS) 48h after the transfection. Statistical results of the mean fluorescence intensity (MFI) of positive cells are shown in FIG. 3.

In FIG. 3, an mRNA row represents the BFP reporter system plasmid added to the corresponding well, and an arRNA row represents the arRNA added to the corresponding well. In the BFP reporter system, the three bases at the 198th, 199th, and 200th positions are CCA in the original sequence, and while C at the 198th position is changed to A, T or G, the corresponding amino acids at the 65 position are all threonines, so the change of the 198th position of the four different reporter systems BFP-GCA, BFP-CCA, BFP-ACA and BFP-TCA may not cause the change of the original protein function. While C at the 199th position is edited to U, the codons formed by bases at the 199th, 200th, and 201th positions are changed from CAC to UAC, and the corresponding amino acid at the 66th position is changed from a histidine (His, H) to a tyrosine (Tyr, Y), thereby the fluorescence transition of BFP to GFP is achieved. As shown in FIG. 3, while any arRNAs are not added, the background GFP signal MFI of the reporter system is about 5 × 10⁴ (the reporter system in which the mRNA row is marked as U^{∗}, the arRNA row is marked as /; and the reporter system in which the mRNA row is marked as A^{∗}, and the arRNA row is marked as /). However, while the C at the 199th position is mutated to T by a point mutation at the DNA level, the GFP signal MFI is about 2.4×10⁶~3.1×10⁶, and is about 100 times higher than a background value. Therefore, if the C at the 199th position is all changed to U at the RNA level, it may result in an approximately 100-fold increase in GFP MFI.

On the basis that the C at the 199th position at the DNA level remains unchanged, as shown in FIG. 3, while arRNA is added, MFI of GFP may be increased to more than 5 × 10⁵ at most, and the fluorescence intensity exceeds 20% of the fluorescence intensity after the C at the 199th position is changed to T at the DNA level. It is indicated that the technology of the present application may change the final protein function by converting the C at the 199th position to U at the transcriptional level without changing the DNA sequence.

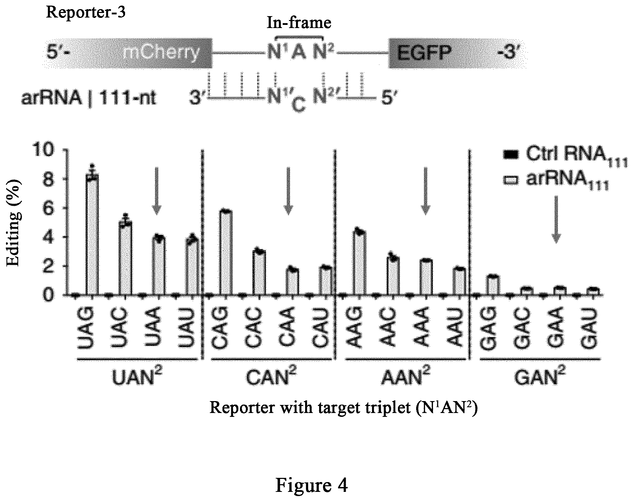

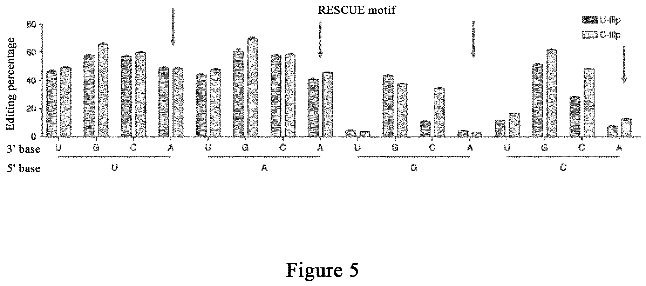

In an LEAPER technical document (Qu et al., 2019, original figure 2f, corresponding to FIG. 4 of the present application), while the 3' adjacent base of the target base (as shown in a N² position) is A, the preference (referring to the editing efficiency obtained by the same arRNA while the 5' upstream base is A, U, G or C) of the LEAPER technology for the 5' adjacent base of the target base (as shown in a N1 position) is: U > C≈A > G. In a RESCUE technical document (Abudayyeh et al., 2019, original figure 1c, corresponding to FIG. 5 in the present application), while the 3' adjacent base of the target base is A, the preference of the RESCUE technology for the 5' adjacent base of the target base is: U≈A >> C≈G. In the CUSPER technology of the present application, it is unexpectedly found that while the 3' adjacent base of the target base is A, the preference for the 5' upstream base of the target base is different from both the LEAPER technology and the RESCUE technology. As shown in FIG. 3, if the fixed arRNA is U^ or C^ with the higher editing efficiency, it may be seen that the preference of the technology of the present application for the 5' upstream base is: G > C >> U≈A.

### 3. Further determination of base preference of CUSPER

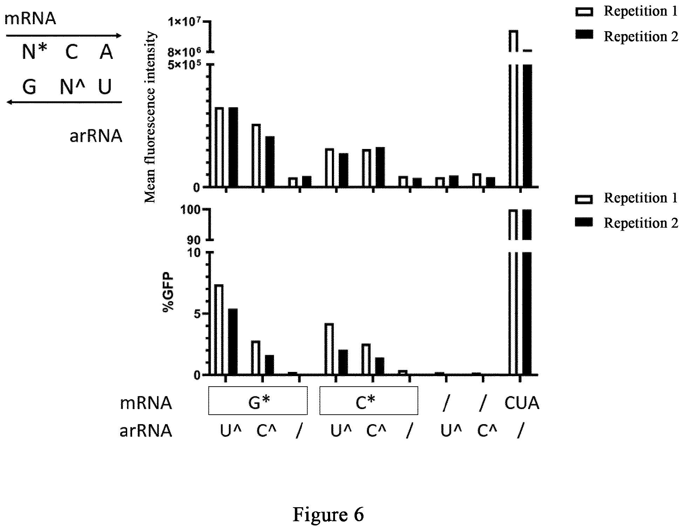

In order to further determine the editing ability of CUSPER and its base preference, a second part of Example 2 is further repeated, as shown in FIG. 6. Compared with the second part of Example 2, this part supplements a control experiment in the case of only two reporter system plasmids, BFP-GCA and BFP-CCA which are not involved in the second part of Example 2, without adding any arRNAs. In addition, conditions in which the MFI exceeds the background value by more than 2 times in the relevant test in FIG. 3 are repeated. At the same time, repetition 1 and repetition 2 in the experiment correspond to two strains of ADAR2-r16-293T produced in different batches.

As shown in FIG. 6, the system's preference for the 5' adjacent bases of the target base in the repeated experiment show a pattern similar to the relevant test of the second part of Example 2 shown in FIG. 3, it has the better editing efficiency while the 5' adjacent base is G or C, and G is greater than C.

The above results show that the technology of the present application may affect the final protein function from the transcriptional level without changing the DNA sequence. At the same time, the preference of this technology for the 5' adjacent bases of the target base may be different from that of the LERPER and RESCUE technologies, and it is shown in Table 2 in detail. It is worth noting that in the tests of BFP-GCA, BFP-TCA, and BFP-ACA in this research, the design of arRNA not only has the mismatch at the target base, but also has another mismatch at the 3' downstream of the arRNA (as shown in FIG. 2). Therefore, it is necessary to further test whether there is a similar pattern in the preference of the technology for the 5' upstream bases while there are no mismatches in these positions.

**Table 2: Preference comparison of adjacent bases of target bases of three RNA editing technologies**

| Technical name | Mismatch number | Preference for 5' adjacent base while 3' adjacent base of target base is A |
|---|---|---|
| CUSPER technology | One mismatch in CCA Two mismatches in others | G > C >> A ≈ U |
| LEAPER ⁴ | One mismatch | U > C ≈ A > G |
| RESCUE ¹ | One mismatch | U≈A >> C≈G |

### Example 3: Test of CUSPER editing system in absence of mismatches of both 3' and 5' adjacent bases

By reading a green fluorescent protein (GFP) signal, the editing efficiency of different arRNAs may be quickly and roughly judged, but if the editing efficiency needs to be further confirmed, Next Generation Sequencing (NGS) is required to finally confirm what proportion of C in mRNA is edited into U. At the same time, in the RNA single-base editing system, whether it is A to I (Qu et al., 2019) or C to U (Abudayyeh et al., 2019), the 5' adjacent base and 3' adjacent base of its target base A or C have the greater impact on its editing efficiency. Due to the limitation of the BFP to GFP reporter system, if the C at the 199th position in the DNA sequence of BFP (SEQ ID NO 2) is used as the target base, while the 5' adjacent base (the 198th position) C is changed to A, T, and G, the corresponding amino acid at the 65th position is not affected; but while the 3' adjacent base (the 200th position) A is changed to T, C or G, it is caused that it completely loses the GFP signal. Therefore, the test of reading GFP signal by the reporter system may not test the system with the 3' adjacent bases as T, C, and G. However, if the NGS is used, the percentage of U in A, U, C, and G in the edited mRNA may be directly read, so that it is more convenient to compare the editing efficiency in a total of 16 cases of permutations and combinations of 4 different 5' adjacent bases and 4 different 3' adjacent bases.

Referring to steps of Example 1.2, according to the different DNA sequences in the 198th, 199th and 200th positions in the DNA sequence of BFP (SEQ ID NO 2), 16 different reporter systems are constructed, namely ACA, ACT (corresponding mRNA: ACU), ACC, ACG, TCA (corresponding mRNA: UCA), TCT (corresponding mRNA: UCU), TCC (corresponding mRNA: UCC), TCG (corresponding mRNA: UCG), CCA, CCT (corresponding mRNA: CCU), CCC, CCG, GCA, GCT (corresponding to mRNA: GCU), GCC, GCG. In addition, referring to lentiviral packaging and infection steps of Example 1.1, 293T is infected, so that it is stably integrated into the 293T cells.

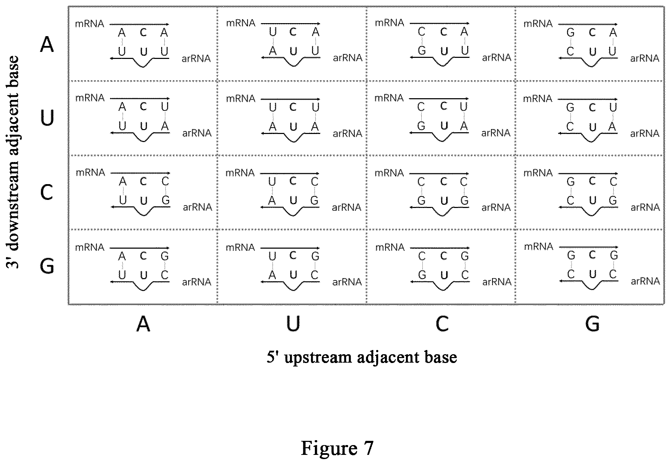

Corresponding to the above 16 different reporter systems, on the basis that the mRNA target base C corresponds to the arRNA targeting base U, the 3' and 5' most adjacent bases of the arRNA targeting base may be complementarily paired with the 5' and 3' adjacent bases of the target base in the target RNA according to a Watson-Crick base pairing principle. The synthesized arRNA sequence is shown in Table 3, wherein a correspondence relationship between the target in the mRNA and its 5' and 3' adjacent bases and the arRNA targeting base and its 5' and 3' most adjacent bases is shown in FIG. 7.

According to the following steps, the corresponding arRNA is transfected into 16 different reporter system cells, and RNA extraction and NGS are performed.
1. A dulbecco's modified eagle medium (DMEM) (Hyclone SH30243.01) comprising 10% fetal bovine serum (FBS) (Vistech SE100-011) is used for cell culture. Reporter system cells are transferred to a 12-well plate at 15,000 cells/well. This time is recorded as 0 hours.
2. 24 hours after cell passage, 12.5 pmol of arRNA is transferred into each well with an RNAi MAX (Invitrogen, 13778150) reagent. The transfection steps are referred to supplier's instructions.
3. 72 hours after cell passage, the whole well of the cells is digested with trypsin (Invitrogen 25300054), a sample is collected with 800 µl TRIzol (Invitrogen, 15596018), and RNA is extracted by using a Direct-zol RNA Miniprep Kit (Zymo Resaerch, R2052). 1000 ng of the extracted total RNA from each sample is taken and a TransScript^{®} One-Step gDNA Removal and cDNA Synthesis SuperMix Kit (TransGen, AT311) is used to perform reverse transcription to synthesize cDNA. 1 µl of a reverse transcription product is taken, and a polymerase chain reaction (PCR) is performed with two primers of which the sequences are ggagtgagtacggtgtgcCTACGGCAAGCTGACCCTGAAGTT (SEQ ID NO: 7) and gagttggatgctggatggGTAGTTGCCGTCGTCCTTGAAGAAG (SEQ ID NO: 8) and Q5 hot start enzyme (NEB, M0494L). PCR products is used to construct a sequencing library with a Hi-TOM kit (NOVOGENE, REF PT045), and NGS and data analysis are completed according to the following steps.

### i. Illumina sequencing

The constructed sequencing library is used for high-throughput sequencing in PE150 mode by a NovaSeq6000 platform.

### ii. Sequencing data processing

The raw data obtained by high-throughput sequencing is quality-controlled with fastp (v0.19.6), and low-quality sequences, sequences with linkers and sequences comprising polyG and the like are filtered out. The obtained high-quality sequencing data is split into each sample according to the corresponding Barcode sequence with a self-developed split script, and BWA (v0.7.17-r1188) software is used to compare with the amplified target area sequence, by SAMtools (v 1.9), the format is converted to generate a BAM file, count comparison information and re-order and build an index.

### iii. Editing efficiency analysis

JACUSA (v1.3.0) software is used to detect all mRNA target bases, parameters used are: call-1 -a B,R,D,I,Y,M:4 -C ACGT -c 2 -p 1 -P UNSTRANDED -R -u DirMult-CE. After filtering out high-frequency mutations that appear in both control and treated samples, three times of the mean mutation frequency other than C->U mutation are used as a threshold, and a part of the mutation frequency of the target bases C to U above the threshold is used as the real frequency at which the target C is mutated to U.

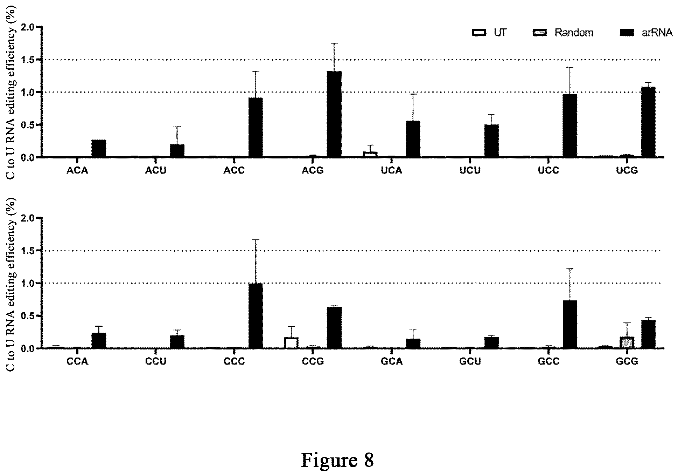

Results of NGS are shown in FIG. 8. It may be confirmed by NGS that the CUSPER system may indeed achieve C-to-U single-base editing of mRNA. Compared with Example 2, it may be seen that if there is no mismatch between the 5' and 3' most adjacent bases of the targeting base and the target RNA, the editing efficiency is relatively low, and while the 5' most adjacent of the targeting base forms a G-G mismatch with the target RNA, the editing efficiency may be improved. In addition, while the 5' and 3' most adjacent bases of the targeting base are all paired with the 3' and 5' adjacent bases of the target base according to the Watson-Crick principle, except for the C-U mismatch of the target base, the higher editing efficiency may be obtained while the target base triplet on the mRNA is the following sequences: ACG, ACC, UCC, UCG, CCC, CCG, UCA, or UCU.

**Table 3: arRNA sequences without mismatches of 3' and 5' adjacent bases**

| arRNA name | arRNA sequence |
|---|---|
| arRNA-GCA | gcugcuucauguggucggguagcggcugaagcacugcacgccgUUCgucaggguggucacgaggg ugggccagggcacgggcagcuugcc （SEQ ID NO: 9） |
| arRNA-GCU | gcugcuucaugugguucgggguagcggcugaagcacugcacgccgAUCgucaggguggucacgaggg ugggccagggcacgggcagcuugcc （SEQ ID NO: 10） |
| arRNA-GCC | gcugcuucauguggucggguagcggcugaagcacugcacgccgGUCgucaggguggucacgaggg ugggccagggcacgggcagcuugcc（SEQ ID NO: 11） |
| arRNA-GCG | gcugcuucaugguggucggguagcggcugaagcacugcacgccgCUCgucaggguggucacgagggu gggccagggcacgggcagcuugcc（SEQ ID NO: 12） |
| arRNA-CCA | gcugcuucaugugguucgggguagcggcugaagcacugcacgccgUUGgucaggugguucacgaggg ugggccagggcacgggcagcuugcc（SEQ ID NO: 13） |
| arRNA-CCU | gcugcuucaugugguucggguagcggcugaagcacugcacgccgAUGgucaggguggucacgaggg ugggccagggcacgggcagcuugcc（SEQ ID NO: 14） |
| arRNA-CCC | gcugcuucauguggucggguagcggcugaagcacugcacgccgGUGgucaggguggucacgaggg ugggccagggcacgggcagcuugcc（SEQ ID NO: 15） |
| arRNA-CCG | gcugcuucaugugguucggguagcggcugaagcacugcacgccgCUGgucaggguggucacgaggg ugggccagggcacgggcagcuugcc（SEQ ID NO: 16） |
| arRNA-UCA | gcugcuucauguggucggguagcggcugaagcacugcacgccgUUAgucaggguggucacgaggg ugggccagggcacgggcagcuugcc（SEQ ID NO: 17） |
| arRNA-UCU | gcugcuucaugugggucggguagcggcugaagcacugcacgccgAUAgucagggugguucacgaggg ugggccagggcacgggcagcuugcc（SEQ ID NO: 18） |
| arRNA-UCC | gcugcuucaugugguucgggguagcggcugaagcacugcacgccgGUAgucaggguggucacgaggg ugggccagggcacgggcagcuugcc（SEQ ID NO: 19） |
| arRNA-UCG | gcugcuucaugguggucggguagcggcugaagcacugcacgccgCUAgucaggguggucacgaggg ugggccagggcacgggcagcuugcc（SEQ ID NO: 20） |
| arRNA-ACA | gcugcuucauguggucggguagcggcugaagcacugcacgccgUUUgucaggguggucacgaggg ugggccagggcacgggcagcuugcc（SEQ ID NO: 21） |
| arRNA-ACU | gcugcuucauguggucggguagcggcugaagcacugcacgccgAUUgucaggguggucacgaggg ugggccagggcacgggcagcuugcc（SEQ ID NO: 22） |
| arRNA-ACC | gcugcuucaugugggucggguagcggcugaagcacugcacgccgGUUgucaggguggucacgaggg ugggccagggcacgggcagcuugcc（SEQ ID NO: 23） |
| arRNA-ACG | gcugcuucaugguggucggggguagcggcugaagcacugcacgccgCUUgucagggugggucacgaggg ugggccagggcacgggcagcuugcc（SEQ ID NO: 24） |
| arRNA-random | uaauccugaauaucgcgcaauuccccagcagagaacaucgcggugugaacgucccuuuauaccgggcag guauagcugaaaucagcguggc（SEQ ID NO: 25） |

| | |
|---|---|
| Note: There is no difference between capital and small letters, and the capital and small letters are only used to highlight differences between the sequences. Wherein arRNA is named by the target base triplet to which it is targeted. | |

### References

1. Abudayyeh, O. O., Gootenberg, J. S., Franklin, B., Koob, J., Kellner, M. J., Ladha, A., ... & Zhang, F. (2019). A cytosine deaminase for programmable single-base RNA editing. Science, 365(6451), 382-386.
2. Cox, D. B., Gootenberg, J. S., Abudayyeh, O. O., Franklin, B., Kellner, M. J., Joung, J., & Zhang, F. (2017). RNA editing with CRISPR-Cas13. Science, 358(6366), 1019-1027.
3. Eckstein, F. (2014). Phosphorothioates, essential components of therapeutic oligonucleotides. Nucleic acid therapeutics, 24(6), 374-387.
4. Merkle, T., Merz, S., Reautschnig, P., Blaha, A., Li, Q., Vogel, P., ... & Stafforst, T. (2019). Precise RNA editing by recruiting endogenous ADARs with antisense oligonucleotides. Nature biotechnology, 37(2), 133.
5. Pollard, K. M., Cauvi, D. M., Toomey, C. B., Morris, K. V., & Kono, D. H. (2013). Interferon-y and systemic autoimmunity. Discovery medicine, 16(87), 123.
6. Qu, L., Yi, Z., Zhu, S., Wang, C., Cao, Z., Zhou, Z., ... & Bao, Y. (2019). Programmable RNA editing by recruiting endogenous ADAR using engineered RNAs. Nature biotechnology, 37(9), 1059-1069.
7. Vu, L. T., Nguyen, T. T. K., Md Thoufic, A. A., Suzuki, H., & Tsukahara, T. (2016). Chemical RNA editing for genetic restoration: the relationship between the structure and deamination efficiency of carboxyvinyldeoxyuridine oligodeoxynucleotides. Chemical biology & drug design, 87(4), 583-593.
8. Xu, L., Wang, J., Liu, Y., Xie, L., Su, B., Mou, D., ... & Zhao, L. (2019). CRISPR-edited stem cells in a patient with HIV and acute lymphocytic leukemia. New England Journal of Medicine, 381(13), 1240-1247.

## Claims

1. An engineered composition or system for ribonucleic acid (RNA) editing, comprising:
1) a modified adenosine deaminase protein or a catalytic domain thereof or a construct expressing the modified adenosine deaminase protein or the catalytic domain thereof, wherein the adenosine deaminase protein or the catalytic domain thereof has the activity of catalyzing cytidine deamination after being modified, and
2) an ADAR-recruiting RNA (arRNA) that recruits the modified adenosine deaminase protein or the catalytic domain thereof to a target RNA, or a construct comprising the arRNA or a coding sequence for the arRNA;
wherein the arRNA comprises a complementary RNA sequence that hybridizes to the target RNA, and the arRNA recruits the adenosine deaminase protein or the catalytic domain thereof to the target RNA, thereby deaminating a target cytidine in the target RNA.

2. The engineered composition or system according to claim 1, wherein the adenosine deaminase is modified by mutation in one or more sites to have the activity of deaminating cytidine which consequently converts to uridine.

3. The engineered composition or system according to claim 2, wherein the adenosine deaminase protein or the catalytic domain thereof is an ADAR2 protein or a homologous protein or catalytic domain thereof.

4. The engineered composition or system according to claim 3, wherein the mutation modification comprises: E488Q/V351G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1.

5. The engineered composition or system according to any one of claims 1-4, wherein the arRNA hybridizes to the target RNA, and a targeting base in the arRNA opposite to the target cytidine is A, U, C, or G.

6. The engineered composition or system according to claim 5, wherein the targeting base is U or C.

7. The engineered composition or system according to any one of claims 1-6, wherein:
the arRNA comprises one or more mismatches in one or more positions corresponding to upstream and/or downstream of the target base of the target RNA.

8. The engineered composition or system according to claim 7, wherein the 3' most adjacent base of the arRNA targeting base forms a mismatch with the target RNA.

9. The engineered composition or system according to claim 8, wherein the arRNA hybridizes to the target RNA, and the 3' most adjacent base of the targeting base forms a G-G mismatch with the target RNA.

10. The engineered composition or system according to any one of claims 1-9, wherein the arRNA hybridizes to the target RNA, and the 5' most adjacent base of the targeting base does not form a mismatch with the target RNA.

11. The engineered composition or system according to claim 10, wherein the 5' most adjacent base of the targeting base is U.

12. The engineered composition or system according to claim 9, wherein the arRNA hybridizes to the target RNA, and an order of base preference from high to low in the target RNA opposite to the 5' most adjacent base of the targeting base of the arRNA is G or C, U or A.

13. The engineered composition or system according to any one of claims 1-6, wherein the arRNA hybridizes to the target RNA, a target base triplet formed by the target base and the 5' and 3' adjacent bases thereof forms a mismatch only at the target base, wherein the target base triplet is selected from: ACG, ACC, UCC, UCG, CCC, CCG, UCA, and UCU.

14. The engineered composition or system according to any one of claims 1-13, wherein the length of the arRNA is >50 nt.

15. The engineered composition or system according to any one of claims 1-14, wherein the lengths from the targeting base to the 3'-terminal and 5'-terminal in the arRNA are equal.

16. The engineered composition or system according to any one of claims 1-14, wherein the length from the targeting base to the 3'-terminal in the arRNA is 45-5 nt, 40-5 nt, 35-10 nt, 25 nt-15 nt, or 24 nt-11 nt.

17. The engineered composition or system according to any one of claims 1-14, wherein the length from the targeting base to the 5'-terminal in the arRNA is 80-30 nt, 70-35 nt, 60-40 nt, 55 nt-35 nt, or 55 nt -45 nt.

18. The engineered composition or system according to any one of claims 1-17, wherein the arRNA is chemically modified.

19. The engineered composition or system according to claim 18, wherein the chemical modification comprises a 2'-O-methyl modification or an inter-nucleotide 3'-thio modification.

20. A method for deaminating a target cytosine in a target RNA in a cell, comprising introducing the followings 1) and 2) into the cell:
1) a modified adenosine deaminase protein or a catalytic domain thereof or a construct expressing the modified adenosine deaminase protein or the catalytic domain thereof, and
2) an arRNA that recruits the modified adenosine deaminase protein or the catalytic domain thereof to the target RNA or a construct comprising the arRNA or a coding sequence for the arRNA, wherein the adenosine deaminase protein or the catalytic domain thereof has the activity of catalyzing cytidine deamination after being modified, and the arRNA comprises a complementary RNA sequence that hybridizes to the target RNA, and the arRNA recruits the adenosine deaminase protein or the catalytic domain thereof to the target RNA, thereby deaminating a target cytidine in the target RNA.

21. The method according to claim 20, wherein the construct expressing the modified adenosine deaminase protein or the catalytic domain thereof and the construct comprising the coding sequence for the arRNA are the same construct, or the construct expressing the modified adenosine deaminase protein or the catalytic domain thereof and the construct comprising the coding sequence for the arRNA are separated constructs, and the separated constructs are introduced into the cell simultaneously or separately.

22. The method according to claim 20 or 21, wherein the adenosine deaminase is modified by mutation in one or more sites to have the activity of deaminating cytidine which subsequently converts to uridine.

23. The method according to claim 22, wherein the adenosine deaminase protein or the catalytic domain thereof is an ADAR2 protein or a homologous protein or catalytic domain thereof.

24. The method according to claim 23, wherein the mutation modification comprises: E488Q/V35 1G/S486A/T375S/S370C/P462A/N597I/L332I/I398V/K350I/M383L/D619G/S58 2T/V440I/S495N/K418E/S661T, wherein the numbering of the amino acid of the mutation modification is consistent with the numbering of the amino acid in NP_001103.1.

25. The method according to any one of claims 21-24, wherein the arRNA hybridizes to the target RNA, and a targeting base in the arRNA opposite to the target cytidine is A, U, C, or G.

26. The method according to claim 25, wherein the targeting base is U or C.

27. The method according to any one of claims 21-26, wherein:
the arRNA comprises one or more mismatches in one or more positions corresponding to upstream and/or downstream of the target base of the target RNA.

28. The method according to claim 27, wherein the 3' most adjacent base of the arRNA targeting base forms a mismatch with the target RNA.

29. The method according to claim 28, wherein the arRNA hybridizes to the target RNA, and the 3' most adjacent base of the targeting base forms a G-G mismatch with the target RNA.

30. The method according to any one of claims 21-29, wherein the arRNA hybridizes to the target RNA, and the 5' most adjacent base of the targeting base does not form a mismatch with the target RNA.

31. The method according to claim 30, wherein the 5' most adjacent base of the targeting base is U.

32. The method according to claim 29, wherein the arRNA hybridizes to the target RNA, and an order of base preference from high to low in the target RNA opposite to the 5' most adjacent base of the targeting base of the arRNA is G or C, U or A.

33. The method according to any one of claims 21-26, wherein the arRNA hybridizes to the target RNA, a target base triplet formed by the target base and the 5' and 3' adjacent bases thereof forms a mismatch only at the target base, wherein the target base triplet is selected from: ACG, ACC, UCC, UCG, CCC, CCG, UCA, and UCU.

34. The method according to any one of claims 21-33, wherein the length of the arRNA is >50 nt.

35. The method according to any one of claims 21-34, wherein the lengths from the targeting base to the 3'-terminal and 5'-terminal in the arRNA are equal.

36. The method according to any one of claims 21-34, wherein the length from the targeting base to the 3'-terminal in the arRNA is 45-5 nt, 40-5 nt, 35-10 nt, 25 nt-15 nt, or 24 nt-11 nt.

37. The method according to any one of claims 21-34, wherein the length from the targeting base to the 5'-terminal in the arRNA is 80-30 nt, 70-35 nt, 60-40 nt, 55 nt-35 nt, or 55 nt -45 nt.

38. The method according to any one of claims 21-37, wherein the arRNA is chemically modified.

39. The method according to claim 38, wherein the chemical modification comprises a 2'-O-methyl modification or an inter-nucleotide 3'-thio modification.

40. The method according to any one of claims 21-39, wherein the cell is a mammalian cell.

41. A method for treating a disease caused by a T-to-C mutation, comprising using the method according to any one of claims 21-39 to deaminate a target base C in a transcription-formed messenger RNA comprising the T-to-C mutation to correct the mutation.

42. A modified adenosine deaminase protein, wherein the adenosine deaminase protein is ADAR2 comprising the following amino acid mutations corresponding to ADAR2 with Genebank accession number NP_001103.1: E488Q/V351G/S486A/T375S/S370C/P462A/N597I/L332I/I398V/K350I/M383L/D619G/S58 2T/V440I/S495N/K418E/S661T, or an amino acid mutation in the corresponding position of a homologous ADAR2 protein of the NP_001103.1, and the ADAR2 protein has the activity of catalyzing cytidine deamination after being modified by the mutation.

43. A use of the modified adenosine deaminase protein according to claim 42 for catalyzing cytidine deamination into uridine.
